# EUROPEAN PATENT APPLICATION

(11) **EP 1 034 786 A1**
(43) Date of publication of application: **13.09.2000**
(21) Application number: 00108537.2
(22) Date of filing: 28.01.1992
(51) Int. Cl.: A61K 35/74, A01K 45/00, A23K 1/00

(54) **Method for delivering direct feed microorganisms to poultry in ovo**

(30) Priority: 28.01.1991 US 646879
(62) Divisional of application: 92906512.6
(71) Applicant: BIOGAIA BIOLOGICS AB, 103 64 Stockholm (SE); CASAS-PEREZ, Ivan A., Raleigh, NC 27612 (US)
(72) Inventor: Casas-Perez, Ivan A., Raleigh, NC 27609 (US); Edens, Frank W., Raleigh, NC 27609 (US)
(74) Representative: Fagerlin, Heléne

(57) **Abstract**

A method of establishing direct feed microorganisms such as *Lactobacillus reuteri* in the gastrointestinal tract of avian organisms in which eggs are inoculated with living cells of the microorganism.

## Description

### FIELD OF INVENTION

This invention relates to a new method for delivering viable Lactobacillus reuteri cells to avian organisms in ovo.

### BACKGROUND INFORMATION

The terms "probiotics" is attributed to Parker (20) who defined them as "organisms and substances which contribute to intestinal balance" when used as dietary supplements. Later, Fuller (11) considered this definition to be too broad since, in addition to including cell cultures and microbial metabolites, it could encompass antibiotic preparations. More recently, a number of summaries have appeared in the literature describing the scientific basis for use of probiotics as intestinal inoculants for production animals (10, 26). It has been suggested that the term "probiotics" be replaced by the term "direct feed microorganisms", or DFM's (9).

The concept of adding viable, harmless lactic acid bacteria to the gastrointestinal tract as a dietary supplement was first appreciated by Metchnikoff (16) who viewed the consumption of yoghurt by Bulgarian peasants as conferring a long span of life. Some workers have claimed that the therapeutic value derived from ingestion of such fermented milk products is related to the viable bacteria present in these products (12, 27). Since Metchnikoff's early reports, several studies have shown the ability of lactobacilli, for example, to suppress coliform growth. Feeding viable Lactobacillus acidophilus cells to young dairy calves was shown to reduce the incidence of diarrhoea (3), and increase the numbers of lactobacilli and reduce coliform counts in feces (4). These findings contrast with those of others who were unable to demonstrate benefits from feeding either Lactobacillus acidophilus (8, 13) or milk cultured with Lactobacillus acidophilus or Lactobacillus lactis (17).

In a detailed study by Muralidhara et al. (18), piglets given a Lactobacillus lactis concentrate for up to 8 weeks after birth showed a progressive decline in coliform counts in fecal samples. Scouring in these animals was negligible, but was evident in control pigs especially at weaning. Underdahl et al. (32) observed only mild diarrhoea lasting 2-4 days in gnotobiotic pigs inoculated with Streptococcus faecium prior to artificial Escherichia coli infection. In the same study, persistent diarrhoea occurred in pigs similarly infected with Escherichia coli, but without prophylactic treatment with the Streptococcus microorganism.

Probiotics (hereafter referred to as DFM's) are bacterial or yeast preparations that are administered orally or added to feeds. The most commonly used DFM's are strains of the lactic acid bacteria (LAB), particularly those classified in the following genera: Lactobacillus, Lactococcus, and Enterococcus. Included among these are the following species: Lactobacillus acidophilus, Lactobacillus bulgaricus, Lactobacillus plantarum, Lactobacillus casei, Lactobacillus lactis, Lactococcus lactis, Lactococcus thermophilus, Lactococcus diacetylactis, and Enterococcus faecium. Besides these LAB, some species of Bacillus (Bacillus subtilis, Bacillus toyoi) and yeasts and molds (Saccharomyces cerevisiae, Aspergillus oryzae, and Torulopsis sp.) are used as DFM's (10).

It is generally held that during periods of low resistance, such as stress, undesirable microorganisms are able to proliferate in the GI tract of animals, humans included. Maintaining a normal, healthy balance of microorganisms is deemed to be critical during such stressful periods (10). The concept underlying use of DFM's, therefore is that if sufficient numbers of an appropriate microorganism(s) are introduced into the intestinal tract (i) at times of stress and/or disease, (ii) at birth, or (iii) after antibiotic treatment (when minimal LAB are present), the negative consequences of the microbial imbalances can be minimized or overcome. Using such preparations of live, naturally occurring microorganisms helps restore and maintain the proper balance of beneficial microbes in the GI tract during times of stress, disease, and following antibiotic therapy (10). This concept, descriptions of proposed modes of action, and evidence for the efficacious uses of DFM's for all production animals are summarized in reviews by Fox (10), Sissons (26), and by various authors (22).

One of the major problems or limitations encountered in commercial scale application of DFM's to animals is (i) the availability of suitable delivery systems, and (ii) the ability to get the probiotic preparations to the animals as quickly as possible after birth. This is particularly true when pelletized feeds are used, as is the case in the poultry industry. The pelletization process generally includes one or more heating steps involving temperatures high enough to pasteurize or sterilize the feed components, thereby precluding incorporation of viable microorganisms into these feeds prior to pelletization.

The present invention describes novel methods and processes for overcoming some of these problems, by delivering viable DFM's in ovo. The DFM used to develop these methods is Lactobacillus reuteri. This species was chosen because it has demonstrated efficacy as a DFM in poultry (21). Previous patent applications have been submitted relating to unique properties of the species. These applications are: PCT/US88/01423, filed April 28, 1988 and published November 3, 1988, USP 5,439,678, and PCT/US91/03796.

Lactobacillus reuteri is a species of lactic acid bacteria recognized since the turn of the century (19). Originally assigned different species names (e.g. Lactobacillus fermentum biotype II), it obtained distinct species status in 1980 and is registered in the 1988 edition of Bergey's manual (14, 15). It is found in foods, particularly dairy products and meats, but exists primarily in the GI tract of healthy animals, including humans (1, 6, 7, 14, 15, 23, 24, 25, 33).

Lactobacillus reuteri is the dominant heterofermentative Lactobacillus inhabiting the GI tract (23, 24, 25). It is a typical heterofermenter, converting sugars into acetic acid, ethanol, and CO₂ in addition to lactic acid which is the major endproduct of homo-fermentative metabolism carried out by species such as Lactobacillus acidophilus (31).

It utilizes the phosphoketolase pathway for conversion of glucose to endproducts. When glycerol, an alternate hydrogen acceptor, is present in the culture medium together with glucose or other utilizable carbon and energy sources (e.g. lactose), acetate rather than ethanol accumulates, and the glycerol is reduced to 1,3-propanediol via the metabolic intermediate, 3-hydroxypropionaldehyde (3-HPA). 3-HPA has been shown to have potent antimicrobial activity, and Lactobacillus reuteri appears to be unique among microorganisms examined to date in its ability to secrete this substance, termed reuterin, into the surrounding medium (2, 5, 7, 28, 29, 30, 31). This unique antimicrobial activity may play a role in competitive survival of this species in the gastrointestinal ecosystem, and/or its ability to regulate growth and activities of other microorganisms in this ecosystem (7). It is thus very important to establish this microorganism early in animals. It is therefore an object of the invention to provide a method for delivering Lactobacillus reuteri to avian species. Other objects and advantages will be more fully apparent from the following disclosure and appended claims.

### SUMMARY OF INVENTION

Pure cultures of Lactobacillus reuteri are injected into eggs with no detrimental effect on their hatchability. The invention generally provides a means for delivering lactobacilli reuteri into eggs of avian species, so that these microorganisms may be well established in the bird gastrointestinal system at hatching time.

WO-A-90/14765 discloses a method of injecting a bacterial culture into the air cell of a bird egg prior to hatching of the egg. The bacteria may be any bacteria. Undefined cultures are preferred. Lactobacillus reuteri is not mentioned. The hatchability is lowered (82%) compared to controls (96%) (see Example 4, Table 3). According to the present invention the hatchability is increased to between 98 and 100% (see Table 1).

WO-A-88/08452 discloses a method of establishing a direct feed microorganism in the gastrointestinal tract of an animal, including birds, by administration of Lactobacillus reuteri.

Neither of these two documents, however, describes a method of injecting eggs with living L. reuteri that does not reduce hatchability.

Other aspects and features of the invention will be more fully apparent from the following disclosure and appended claims.

### DETAILED DESCRIPTION OF THE INVENTION AND PREFERRED EMBODIMENTS THEREOF

The present invention provides a method of providing Lactobacillus reuteri to avian embryos in the eggs so that the L. reuteri are established in the gastrointestinal tract of the newly hatched birds. Using methods of delivery previously developed for delivery of antibiotics (see US Patent Nos. 4,681,063 and 4,903,635, for example) or a manual needle puncture of the egg into the air cell, live cells of L. reuteri are delivered into the air cell in incubating eggs.

The features and advantages of the present invention will be more clearly understood by reference to the following example.

### EXAMPLE

Eggs of turkeys (Nicholas) or chickens are aseptically punctured above the air cell with a needle, preferably about 2 days before hatch. With a syringe and needle, 100 µl of a suspension of Lactobacillus reuteri, strain T-1 (isolated from turkeys) or strain 11284 (isolated from chickens), containing about 10⁵, 10⁷, or 10⁸ cells of the strain, is injected into the air cell. Each of these strains has been deposited with the American Type Culture Collection in Rockville, Maryland. Table 1 shows the effect on hatchability with varying levels of Lactobacillus inoculation. The data presented in Table 1 for turkeys show that pure cultures of Lactobacillus reuteri can be successfully introduced into viable poultry eggs without effecting the hatchability of the eggs. The percentage hatchability was unaffected by this inoculation. Similar results are obtained for chickens.

**Table 1**

| TREATMENT | % LIVE EMBRYOS AT HATCH | % OF SURVIVORS AT DAY 7 |
|---|---|---|
| Untreated embryos | 96 | 81 |
| Phosphate injected (control) | 97 | 81 |
| L. reuteri air cell injected, 10⁵ CFU | 98 | 85 |
| L. reuteri air cell injected, 10⁷ CFU | 100 | 78 |
| L. reuteri air cell injected, 10⁸ CFU | 94 | 83 |

This in ovo method serves as a new means for introducing the defined beneficial microorganism L. reuteri such as a pure strain thereof into the gastrointestinal tract of poultry at an early stage. The embryonic chick or poult is immersed in amniotic fluid which is in contact with the gastrointestinal tract. Thus, the microorganism inoculated in ovo can become established in the bird's gastrointestinal tract.

The data presented in Table 2 show that the birds thus inoculated in ovo with Lactobacillus reuteri in fact have this microorganism in their gastrointestinal tract when they hatch. In this example the total number of lactobacilli found in the bird's cecum was determined for each treatment. Also determined was the percent of these lactobacilli which were L. reuteri as identified by this species' ability to produce the inhibiting agent, reuterin.

The number of total lactobacilli present was determined as colony forming units (CFU) per excised and homogenized cecum using solid (1.5% Agar) Lactobacillus Selection Medium (LBS) as described in references 2, 5, and 7. The percent of these colonies which were L. reuteri was determined as described in international patent application PCT/US88/01423, but using L. plantarum as the indicator organism. In this test, colonies of lactobacilli on the LBS agar medium are overlaid with 10 ml of 1% liquified agar containing 0.5 M glycerol and a L. plantarum inoculum. After anaerobic (Gas-Pack System) incubation at 37°C for 24 hours, zones of growth inhibition are seen around colonies that produce reuterin from glycerol. These colonies are thus identified and enumerated as L. reuteri.

Table 2 shows the total lactobacilli found at hatch and the percent of these that were L. reuteri. It can be seen in column 1 of Table 2 that control treatments (untreated and phosphate injected) yielded hatchlings with no L. reuteri in their ceca although a few other lactobacilli could be found. When the treatments included the different inoculum levels of L. reuteri, this microorganism could be found in all the ceca, constituting 33% of the colonies isolated.

**Table 2**

| TREATMENT | Total | % L. reuteri |
|---|---|---|
| Untreated embryos | 3.3x10² | 0 |
| | | |
| Phosphate injected | <5.0x10¹ | 0 |
| | | |
| L. reuteri (10⁵ CFU), | 3.3x10⁵ | 33 |
| air cell injected | | |
| | | |
| L. reuteri (10⁷ CFU), | 1.2x10⁶ | 33 |
| air cell injected | | |
| | | |
| L. reuteri (10⁸ CFU), | 4.7x10⁵ | 33 |
| air cell injected | | |

While the invention has been described with reference to specific embodiments thereof, it will be appreciated that numerous variations, modifications, and embodiments are possible, and accordingly all such variations, modifications, and embodiments are to be regarded as being within the spirit and scope of the invention.

### BEST MODE FOR CARRYING OUT THE INVENTION

Pure cultures of Lactobacillus reuteri are injected into eggs by puncturing the egg aseptically above the air cell with a needle. The invention generally provides a means for delivering Lactobacillus reuteri into eggs of avian species, so that these microorganisms may be well established in the bird gastrointestinal system at hatching time.

### INDUSTRIAL APPLICABILITY

This invention provides an in ovo method for introducing the defined beneficial microorganism Lactobacillus reuteri such as a pure strain thereof into the gastrointestinal tract of poultry at an early stage. This results in enhanced protection from pathogenic microorganisms and increased weight gain for poultry, such as chickens and turkeys.

### REFERENCES

1. Axelsson L, Lindgren SE. 1987. Characterization and DNA homology of Lactobacillus reuteri strains isolated from pig intestine. J. Appl. Bacteriol., 62:433-440.
2. Axelsson L, Chung TC, Dobrogosz WJ, Lindgren SE. 1989. Production of a broad spectrum antimicrobial substance by Lactobacillus reuteri. Microbial Ecol. Health Dis., 2:131-136.
3. Bechman TL, Chambers JV, Cunningham MD. 1977. Influence of Lactobacillus acidophilus on performance of young dairy calves. J. Dairy Sci., 60:74(abs).
4. Bruce BB, Gilliland SE, Bush LJ, Staley TE. 1979. Influence of feeding cells of Lactobacillus acidophilus on the fecal flora of young calves. Oklahoma Anim. Sci. Res. Rep., 207.
5. Chung TC, Axelsson L, Lindgren SE, Dobrogosz WJ. 1989. In vitro studies on reuterin synthesis by Lactobacillus reuteri. Microbial Ecol. Health Dis., 2:137-144.
6. Dellaglio F, Arrizza FS, Leda A. 1981. Classification of citrate fermenting lactobacilli isolated from lamb stomach, sheep milk and pecorino romano cheese. Zbl. Bakt. Hyg., Abt. Orig. C2:349-356.
7. Dobrogosz WJ, Casas IA, Pagano GA, Talarico TL, Sjorberg B-M, Karlson M. 1989. Lactobacillus reuteri and the enteric microbiota. In: The Regulatory and Protective Role of the Normal Microflora (Eds: GrubbR, MidtvedtT, NorinE.) Macmillan LTD, London, pp. 283-292.
8. Ellinger DK, Muller LD, Gantz PJ. 1978. Influence of feeding fermented colostrum and Lactobacillus acidophilus on fecal flora and selected blood parameters of young dairy calves, J. Dairy Sci., 61:162(abs).
9. Food and Drug Administration Compliance Policy Guide No. 7126.41, May 2, 1988.
10. Fox SM. 1988. Probiotics: Intestinal inoculants for production animals. Food-Animal Practice, Vet. Med., August issue.
11. Fuller R. 1986. Probiotics. J. Appl. Bacteriol. Symp. Suppl., 1S-7S.
12. Goodenough ER, Kleyn DH. 1976. Influence of viable yoghurt microflora on the digestion of lactose by the rat. J. Dairy Sci., 59:601-606.
13. Hatch RC, Thomas RO, Thayne WV. 1973. Effect of adding Bacillus acidophilus to milk fed to baby calves. J Dairy Sci., 56:682(abs).
14. Kandler O, Stetter K, Kohl R. 1980. Lactobacillus reuteri sp. nov. a new species of heterofermentative lactobacilli. Zbl. Bakt. Hyg. Abt. Orig. Cl:264-269.
15. Kandler O, Weiss N, 1986. Regular nonsporing Gram positive rods. Bergey's Manual of Systematic Bacteriology (Eds.: Sneath DHA, Mair NC, Sharpe ME, Holt JH), vol. 2:1208-1234. Williams and Wilkins, NY.
16. Metchnikoff E. 1907. Prolongation of Life. Heinemann, London.
17. Morrill JL, Dayton AD, Mickelson R. 1977. Cultures milks and antibiotics for young calves. J. Dairy Sci., 60:1105.
18. Muralidhara KS, Sheggeby GG, Elliker PR, England DC, Sandine WE. 1977. Effects of feeding lactobacilli on the coliform and Lactobacillus flora of intestine tissue and feces from piglets. J. Food Protection, 40:288-295.
19. Orla-Jensen S. 1943. The lactic acid bacteria. Det Kongelige Danske Videnskabernes Selskab. Biologiske Skrifter, Bind II, Nr 3. Kobenhavn.
20. Parker RB. 1974. Probiotics, the other half of the antibiotic story. Anim. Nutr. Health. 29:4-8.
21. Parkhurst CR, Edens FW, Casas IA. 1991. Lactobacillus reuteri and whey reduce Salmonella colonization in turkey poults. International Poultry Trade Show, Southeastern Poultry and Egg Association, Atlanta, GA, Abs. Sci. Meet., Jan. 30 - Feb. 1, 1991.
22. REVUE: Scientifique et Technique, Digestive Microflora and Bioregulation, International Office of Epizootics, F-75017, Paris, France, Vol. 8, June, 1989.
23. Sarra PG, Magri M, Bottazzi V, Dellaglio F, Bosi E. 1979. Frequenza di bacilli heterofementanti nelle feci di vitelli lattanti. Arch. Vet. Ital., 30-16-21.
24. Sarra PG, Dellaglio F, Battazzi V. 1985. Taxonomy of lactobacilli isolated from the alimentary tract of chickens. System. Appl. Microbiol., 6:86-89.
25. Sarra PG, Vescovo M, Fulgoni M. 1986. Study on crop adhesion genetic determinant in Lactobacillus reuteri. Microbiologica, 9:279-285.
26. Sissons JW. 1989. Potential of probiotic organisms to prevent diarrhoea and promote digestion in farm animals - a review. J. Sci. Food Agric., 46:1-13.
27. Speck ML. 1977. Heated yoghurt - is it still yoghurt? J. Food Protection. 40:863-865.
28. Talarico TL, Casas IA, Chung TC, Dobrogosz WJ. 1988. Production and isolation of reuterin: a growth inhibitor produced by Lactobacillus reuteri. Antimecrob. Agents Chemotherap., 32:1854-1858.
29. Talarico TL, Dobrogosz WJ. 1989. Chemical characterization of an antimicrobial substance produced by Lactobacillus reuteri. Antimicrob. Agents Chemotherap., 33:674-679.
30. Talarico TL, Dobrogosz WJ. 1990. Purification and characterization of glycerol dehydratase from Lactobacillus reuteri. Appl. Environ. Microbiol., 56:1195-1197.
31. Talarico TL, Axelsson L, Novotny J, Fiuzat M, Dobrogosz WJ. 1990. Utilization of glycerol as a hydrogen acceptor by Lactobacillus reuteri: Purification of 1,3-propanediol:NAD oxidoreductase. Appl. Environ. Microbiol., 56:943-948.
32. Underdahl NR, Torres-Medina A, Doster AR. 1982. Effect of Streptococcus faecium C-68 in control of Escherichia coli-induced diarrhoea in gnotobiotic pigs. Amer. J. Vet. Res., 43:2227-2232.
33. Vescovo M, Morelli L, Cocconcelli PS, Bottazzi V. 1984. Protoplast formation, regeneration, and plasmid curing in Lactobacillus reuteri. FEMS Microbiol. Lett., 23:333-334.

## Claims

1. A method of establishing a direct feed of Lactobacillus reuteri in the gastrointestinal tract of an avian organism, comprising injecting living cells of the microorganism into an egg of the avian organism prior to hatching of the egg.

2. A method of establishing a direct feed of Lactobacillus reuteri in the gastrointestinal tract of an avian organism, comprising injecting living cells of the microorganism into an egg of the avian organism prior to hatching of the egg with the exception of the purpose to increase the body weight.

3. A method according to claim 1 or 2, wherein the avian organism is a chicken.

4. A method according to claim 1 or 2, wherein the avian organism is a turkey.

5. A method according to claim 1 or 2, wherein the number of microorganisms added to each egg is between 10⁵ and 10⁸ CFU.

6. A method according to claim 1 or 2, wherein Lactobacillus reuteri is injected into the air cell of an egg of the avian organism.
